# EUROPEAN PATENT APPLICATION

(11) **EP 4 439 492 A1**
(43) Date of publication of application: **02.10.2024**
(21) Application number: 24165763.4
(22) Date of filing: 25.03.2024
(51) Int. Cl.: G06V 20/59, A61B 5/16, A61B 5/18, G06F 3/01, G06V 40/18

(54) **ENTROPY OF PUPIL DYNAMICS FOR ESTIMATING ATTENTION TUNNELING**

(30) Priority: 28.03.2023 US 202363455100 P; 20.03.2024 US 202418611304
(71) Applicant: Rockwell Collins, Inc., Cedar Rapids, IA 52498 (US)
(72) Inventor: WU, Peggy, Duluth, GA, 30096 (US); SUNDARAMOORTHI, Ganesh, East Hartford, CT, 06118 (US); RADLBECK, Andrew, uth Glastonbury, CT, 06073 (US); BLAKESLEE, Brigid A., Hamden, CT, 06517 (US)
(74) Representative: Dehns

(57) **Abstract**

An on aircraft computer system records and analyzes biometric data to identify indicia of impaired performance, such as pilot fatigue, attention tunneling, or cognitive overload. Such impairment is identified by alterations in pilot gaze or eye movement, head movement, facial parameters, eye lid position, heart rate, breathing, or brain wave patterns. Appropriate corrective action is applied based on the type of impaired performance identified, including altering a level of automation, contacting a ground dispatcher or ground pilot, or contacting a co-pilot or other crew member. Biometric data is continuously logged and correlated with data from other avionics systems to refine formulas relating biometric data to states of alertness and crew rest procedures.

## Description

### PRIORITY

The present application claims the benefit under 35 U.S.C. § 119(e) of U.S. Provisional App. No. 63/455,100 (filed March 28, 2023).

### BACKGROUND

Pilots are subject to many factors that can impair performance, such as fatigue, cognitive overload, and attention tunneling. Fatigue is a common issue during both long and short haul flights, due to long operating hours, high and stressful workloads, jet lag, short turnaround times, and other factors. Fatigue reduces pilot alertness and reaction times, and results in failure to monitor flight critical information. The current approach to monitor pilot fatigue is based on best practices and self-assessment rather than independent measurement.

Attention tunneling is the involuntary fixation on an information source, which results in a pilot's failure to monitor other information sources. Attention tunneling can be attributed to high or unusual workload environments, automation induced complacency, display location, and other factors.

Consequently, it would be advantageous if a device existed that is suitable for biometrically monitoring pilots and co-pilots to identify attention tunneling.

### SUMMARY

In one aspect, embodiments of the inventive concepts disclosed herein are directed to an on-aircraft computer system that records eye tracking data. The system identifies movements in the eye tracking data indicative of involuntary eye movement. Involuntary eye movements distinguish active focus derived from pilot concentration and inactive attention tunneling.

It is to be understood that both the foregoing general description and the following detailed description are exemplary and explanatory only and should not restrict the scope of the claims. The accompanying drawings, which are incorporated in and constitute a part of the specification, illustrate exemplary embodiments of the inventive concepts disclosed herein and together with the general description, serve to explain the principles.

### BRIEF DESCRIPTION OF THE DRAWINGS

The numerous advantages of the embodiments of the inventive concepts disclosed herein may be better understood by those skilled in the art by reference to the accompanying figures in which:
FIG. 1 shows a block diagram of a system suitable for implementing embodiments of the incentive concepts disclosed herein;
FIG. 2 shows a flowchart of an exemplary embodiment of the inventive concepts disclosed herein.

### DETAILED DESCRIPTION

Before explaining at least one embodiment of the inventive concepts disclosed herein in detail, it is to be understood that the inventive concepts are not limited in their application to the details of construction and the arrangement of the components or steps or methodologies set forth in the following description or illustrated in the drawings. In the following detailed description of embodiments of the instant inventive concepts, numerous specific details are set forth in order to provide a more thorough understanding of the inventive concepts. However, it will be apparent to one of ordinary skill in the art having the benefit of the instant disclosure that the inventive concepts disclosed herein may be practiced without these specific details. In other instances, well-known features may not be described in detail to avoid unnecessarily complicating the instant disclosure. The inventive concepts disclosed herein are capable of other embodiments or of being practiced or carried out in various ways. Also, it is to be understood that the phraseology and terminology employed herein is for the purpose of description and should not be regarded as limiting.

As used herein a letter following a reference numeral is intended to reference an embodiment of the feature or element that may be similar, but not necessarily identical, to a previously described element or feature bearing the same reference numeral (e.g., 1, 1a, 1b). Such shorthand notations are used for purposes of convenience only, and should not be construed to limit the inventive concepts disclosed herein in any way unless expressly stated to the contrary.

Further, unless expressly stated to the contrary, "or" refers to an inclusive or and not to an exclusive or. For example, a condition A or B is satisfied by anyone of the following: A is true (or present) and B is false (or not present), A is false (or not present) and B is true (or present), and both A and B are true (or present).

In addition, use of the "a" or "an" are employed to describe elements and components of embodiments of the instant inventive concepts. This is done merely for convenience and to give a general sense of the inventive concepts, and "a' and "an" are intended to include one or at least one and the singular also includes the plural unless it is obvious that it is meant otherwise.

Finally, as used herein any reference to "one embodiment," or "some embodiments" means that a particular element, feature, structure, or characteristic described in connection with the embodiment is included in at least one embodiment of the inventive concepts disclosed herein. The appearances of the phrase "in some embodiments" in various places in the specification are not necessarily all referring to the same embodiment, and embodiments of the inventive concepts disclosed may include one or more of the features expressly described or inherently present herein, or any combination of sub-combination of two or more such features, along with any other features which may not necessarily be expressly described or inherently present in the instant disclosure.

Broadly, embodiments of the inventive concepts disclosed herein are directed to an on-aircraft computer system that records eye tracking data. The system identifies movements in the eye tracking data indicative of involuntary eye movement. Involuntary eye movements distinguish active focus derived from pilot concentration and inactive attention tunneling.

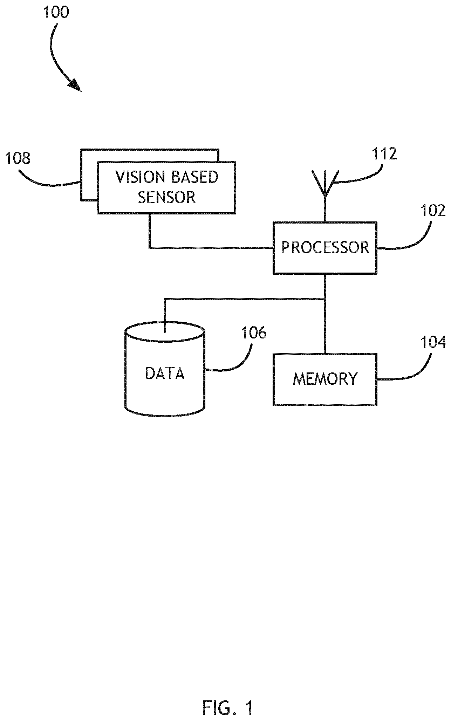

Referring to FIG. 1, a block diagram of a system 100 suitable for implementing embodiments of the incentive concepts disclosed herein is shown. The system 100 includes a processor 102, memory 104 in data communication with the processor 102 for storing processor executable code, and one or more eye tracking sensors / cameras 108 for receiving eye tracking data stream.

In at least one embodiment, the eye tracking sensors 108 record eye movement / gaze of a pilot and eye lid position. The processor executable code configures the processor 102 to continuously log the eye tracking data in a data storage element 106. The processor executable code configures the processor 102 to analyze the eye tracking data to identify small involuntary eye movements that occur in spite of active, intentional fixation. In at least one embodiment, the processor 102 determines if the small involuntary eye movements exceed some minimum threshold indicative of active, intentional fixation; if not, the processor may execute some remedial action such as alerting the user / pilot. Alternatively, or in addition, the processor 102 may alert a remote party such as ground control personnel via a wireless communication device 112.

In at least one embodiment, the processor 102 may compare the small involuntary eye movements to stored profiles. Such profiles may be specific to the user and indicate a user specific minimum threshold.

In at least one embodiment, the processor 102 transfers the stored eye tracking data and other correlated system and task data to an offline storage device for later analysis and correlation to historic data and other outside factors such as crew rest, crew sleep rhythms, flight schedules, etc. Such transfer may be in real time via the wireless communication device 112.

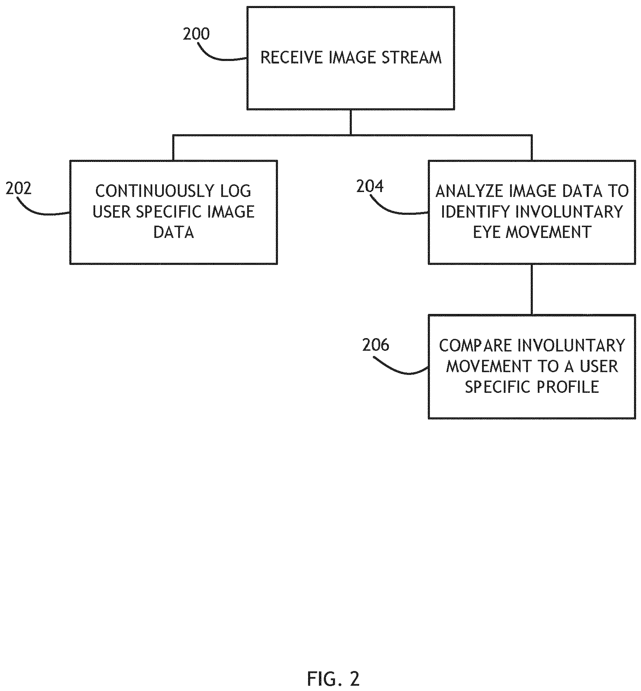

Referring to FIG. 2, a flowchart of an exemplary embodiment of the inventive concepts disclosed herein is shown. A computer system implementing embodiments of the inventive concepts disclosed herein receives 200 an image stream corresponding to eye tracking data from one or more vision based sensors. The eye tracking data is continuously logged 202 and correlated to a specific flight task or an individual duty schedule of the user / pilot.

In at least one embodiment, the eye tracking data is analyzed 204 to identify small involuntary eye movements. A lack of such small eye involuntary eye movement, or incidence below a minimum threshold, may indicate impaired performance or attention tunneling. Small involuntary eye movements may be correlated with eye lid movement / position to further characterize the state of the user / pilot.

In at least one embodiment, the small involuntary eye movements (and potentially eye lid movement / position) may be compared 206 to a stored user specific profile. Recorded small involuntary eye movements and eye lid movement / position may be recorded and used to refine such user specific profiles.

In a situation where attention tunneling is identified, specific instrument cues may be utilized to redirect the pilot's attention. In at least one embodiment, such cues may be organized according to an idealized instrument observation pattern to facilitate the periodic observation of critical instruments. Attention capture techniques such as colors, symbology, blinking or flashing indicators, motion, haptic feedback, or sound may be used to disengage attention tunneling habits and shift the pilot's gaze toward information critical to the operational scenario. Where applicable, eye tracking may be used to determine exactly where the pilot's gaze is focused, and display warning messages at the identified location.

Embodiments of the inventive concepts disclosed herein are critical to enabling reduced crew or single pilot operations, and will provide independent measures necessary to facilitate reduced crew in the cockpit by providing a means to identify when crew members are unable to continue safe flight and notify relief crew or activate automation. Furthermore, a training application may utilize embodiments of the inventive concepts to compare the small involuntary eye movements of a pilot-in-training to previously characterized professional pilot data patterns.

It is believed that the inventive concepts disclosed herein and many of their attendant advantages will be understood by the foregoing description of embodiments of the inventive concepts disclosed, and it will be apparent that various changes may be made in the form, construction, and arrangement of the components thereof without departing from the broad scope of the inventive concepts disclosed herein or without sacrificing all of their material advantages; and individual features from various embodiments may be combined to arrive at other embodiments. The form herein before described being merely an explanatory embodiment thereof, it is the intention of the following claims to encompass and include such changes. Furthermore, any of the features disclosed in relation to any of the individual embodiments may be incorporated into any other embodiment.

## Claims

1. A system comprising:
at least one eye tracking camera; and
at least one processor (102) in data communication with the at least one eye tracking camera and a memory storing processor executable code to configure the at least one processor (102) to:
receive an image stream from the at least one eye tracking camera;
identify involuntary eye movements from the image stream; and
determine if the involuntary eye movements fail to exceed a minimum threshold for a period of time.

2. The system of Claim 1, wherein the at least one processor (102) is further configured to initiate a remedial action, and optionally
wherein the remedial action comprises specific instrument cues to redirect a pilot's attention.

3. The system of Claim 1 or 2, wherein:
the at least one processor (102) is further configured to receive a task or user specific profile of involuntary eye movements; and
the user specific profile defines the minimum threshold.

4. The system of Claim 3, further comprising a data storage element in data communication with the at least one processor (102), wherein the at least one processor (102) is further configured to:
continuously store the identified involuntary eye movements; and
refine the user specific profile, and optionally wherein the at least one processor (102) is further configured to:
record system and task data contemporaneously with the identified involuntary eye movements; and
correlate the system and task data with the identified involuntary eye movements.

5. The system of any preceding Claim, wherein the involuntary eye movements comprise eye lid movement and position.

6. A method comprising:
receiving an image stream from at least one eye tracking camera;
identifying involuntary eye movements from the image stream;
determining if the involuntary eye movements fail to exceed a minimum threshold for a period of time; and
initiating a remedial action.

7. The method of Claim 6, wherein the remedial action comprises rendering specific instrument cues to redirect a pilot's attention.

8. The method of Claim 7, wherein the specific instrument cues are rendered to include attention capturing techniques.

9. The method of any of Claims 6 to 8, further comprising receiving a task or user specific profile of involuntary eye movements, wherein the user specific profile defines the minimum threshold.

10. The method of Claim 9 further comprising:
continuously storing the identified involuntary eye movements; and
refining the user specific profile, and optionally further comprising:
recording system and task data contemporaneously with the identified involuntary eye movements;
correlating the system and task data with the identified involuntary eye movements; and
analyzing the correlated system and task data and identified involuntary eye movements with respect to crew rest, crew sleep rhythms, and flight schedules to refine the minimum threshold.

11. The method of any of Claims 8 to 10, wherein the involuntary eye movements comprise eye lid movement and position.

12. A system for use on an aircraft comprising:
at least one eye tracking camera; and
at least one processor (102) in data communication with the at least one eye tracking camera and a memory storing processor executable code to configure the at least one processor (102) to:
receive an image stream of a pilot from the at least one eye tracking camera;
identify involuntary eye movements from the image stream;
determine if the involuntary eye movements fail to exceed a minimum threshold for a period of time; and
initiate a remedial action.

13. The system of Claim 12, wherein the remedial action comprises specific instrument cues to redirect the pilot's attention.

14. The system of Claim 12 or 13, wherein:
the at least one processor (102) is further configured to receive a task or user specific profile of involuntary eye movements; and
the pilot specific profile defines the minimum threshold, and optionally further comprising a data storage element in data communication with the at least one processor (102), wherein the at least one processor (102) is further configured to:
continuously store the identified involuntary eye movements; and
refine the pilot specific profile, and optionally wherein the at least one processor (102) is further configured to:
record system and task data contemporaneously with the identified involuntary eye movements;
correlate the system and task data with the identified involuntary eye movements;
analyze the correlated system and task data and identified involuntary eye movements with respect to crew rest, crew sleep rhythms, and flight schedules to refine the minimum threshold.

15. The system of any of Claims 12 to 14, wherein the involuntary eye movements comprise eye lid movement and position.
